# EUROPEAN PATENT SPECIFICATION

(11) **EP 3 948 265 B1**
(45) Date of publication and mention of the grant of the patent: **30.04.2025**
(21) Application number: 20784033.1
(22) Date of filing: 25.03.2020
(51) Int. Cl.: G01N 31/22, A61L 2/28, A61L 2/20, A61L 2/07, A61L 2/14

(54) **STERILIZATION ASSESSMENT METHODS WITH CHEMICAL INDICATORS**
STERILISATIONSBEURTEILUNGSVERFAHREN MIT CHEMISCHEN INDIKATOREN
PROCÉDÉS D'ÉVALUATION DE STÉRILISATION AU MOYEN D'INDICATEURS CHIMIQUES

(30) Priority: 29.03.2019 US 201962826694 P
(43) Date of publication of application: 09.02.2022
(73) Proprietor: Advanced Sterilization Products, Inc., Irvine, California 92618 (US)
(72) Inventor: FRYER, Benjamin M., Irvine, California 92618 (US); TRUONG, Doug V., Irvine, California 92618 (US); FRYER, Raya, Lake Forest, California 92630 (US)
(74) Representative: Regimbeau
(86) International application number: PCT/IB2020/052832
(87) International publication number: WO 2020/201933

(56) References cited:
- WO-A1-01/10473
- WO-A2-2012/012055
- WO-A2-2017/044906
- JP-A- 2003 505 106
- KR-A- 20170 102 828
- US-A- 5 916 816
- US-A1- 2011 275 159
- US-A1- 2017 304 476

## Description

### FIELD

The subject matter disclosed herein relates sterilization indicators, particularly chemical indicators that may be used to assess the exposure of a sterilant during a chemical-vapor sterilization procedure.

### BACKGROUND

Medical devices are typically sterilized before use in order to minimize the likelihood that a contaminated device might be used on a subject, which could cause an infection in the subject. Various sterilization techniques may be employed using various sterilizing fluids, often referred to as sterilants, such as steam, hydrogen peroxide, ethylene oxide (EtO), and vapor phase sterilization, either with or without a gas plasma. Each of these techniques depends to a certain extent on the diffusion rates of the sterilization fluids, typically gases, upon the medical devices to be sterilized.

Before sterilization, medical devices are typically packaged within containers or pouches having a semi-permeable barrier that allows transmission of the sterilant, but prevents admission of contaminating organisms, particularly post-sterilization and until the package is opened by medical personnel. For the sterilization cycle to be efficacious, the contaminating organisms within the package must be killed because any organisms that survive the sterilization cycle could multiply and re-contaminate the medical device.

Although the packaging helps prevent contamination of a sterile medical device, the packaging may increase the difficulty of achieving a successful sterilization cycle because the packaging impedes the sterilant from reaching the device or instrument contained therein. This is particularly problematic for devices and instruments that have diffusion-restricted spaces therein because these diffusion-restricted spaces reduce the likelihood that a sterilization cycle may be effective. For example, endoscopes typically have long narrow lumens into which the sterilant must diffuse in sufficient concentration for sufficient time to achieve a successful sterilization cycle.

Confirming that a sterilization cycle has been efficacious helps medical personnel avoid using a contaminated medical device on a subject. Typically, the sterilized medical device is not itself checked for contaminating organisms because such an activity would introduce other contaminating organisms to the medical device, thereby re-contaminating it. Thus, an indirect check has been developed in the form of a sterilization indicator.

A sterilization indicator is a device that may be placed alongside or in proximity to a medical device being subject to a sterilization cycle, such that the sterilization indicator is subject to the same sterilization cycle as the medical device. One type of sterilization indicator is a chemical indicator. Chemical indicators typically comprise a substrate or support upon which is disposed a chemical indicator composition (ink) for detecting an oxidizing agent, such as hydrogen peroxide, which achieves a distinct range of different color changes upon exposure to different doses of the oxidizing agent. Applicant, i.e., Advanced Sterilization Products ("ASP"), Division of Ethicon US, LLC, a Johnson & Johnson company, currently sells chemical indicators, such as the STERRAD^{®} Chemical Indicator Strip, Part no. 14100.

Chemical indicators are classified according to intended applications according to ISO11140, from class 1 to class 6. Of relevance here are class 5 chemical indicators, which have performance comparable to biological indicators, such as ASP's commercially available STERRAD VELOCITY^{™} Biological Indicator, part no. 43210. The STERRAD VELOCITY^{™} Biological Indicator includes a class 5 chemical indicator adhered to an outer surface, which is currently used to provide a user with a quick indication of whether the BI has been exposed to hydrogen peroxide prior to subjecting the biological indicator to further analysis, which could take up to half an hour, or longer.

WO2017/044906 A1 describes a sterilization enclosure comprising one or more sensors for measuring characteristics within the container during a sterilization process, including one or more characteristics of sterilization agent(s), to determine whether instruments disposed within the container have been exposed to threshold process conditions to ensure a desired level of sterilization for those instruments.

WO 01/10473 A1 describes a print on demand sterilization indicator having sterilizing agent sensitive indicia is described. The indicator allows a sterilization cycle to be monitored without the need for a user to subjectively distinguish between color, quality or intensity of display patterns.

WO2012/012055 A2 describes a method for monitoring a sterilization process. The method comprises: exposing an article to be sterilized and a biological indicator to a sterilization medium during a sterilization process, the biological indicator comprising a cell with a plasma membrane; and measuring the membrane potential of the cell to detect the viability of the cell.

### SUMMARY OF THE Invention

According to the present invention, there is provided a method of assessing the efficacy of a sterilization procedure as set out in the independent claims appended hereto. Other features and examples are set out in the corresponding dependent claims. The claimed invention can be better understood in view of the embodiments described hereafter. In general, the described embodiments describe preferred embodiments of the invention. The attentive reader will note, however, that some aspects of the described embodiments extend beyond the scope of the claims. To the respect that the described embodiments indeed extend beyond the scope of the claims, the described embodiments are to be considered supplementary background information and do not constitute definitions of the invention per se. This also holds for the subsequent "brief description of the drawings" and "modes of carrying out the invention".

The present invention relates to a method of assessing an efficacy of a sterilization procedure performed by a sterilization apparatus comprising a chamber. The method comprises placing a chemical indicator in the chamber, commencing a sterilization procedure, obtaining an image of the chemical indicator using a digital imaging device, determining a color value of the image of the chemical indicator, comparing the color value to a color-value threshold, determining that the color value passed the color-value threshold, and automatically ending the sterilization procedure following a determination that the color value has passed the color-value threshold. The method may also include steps of placing an instrument in a non-sterile state in the chamber proximate to the chemical indicator before commencing the sterilization procedure, opening a door of the chamber after the sterilization procedure has ended, and removing the instrument in a sterile state from the chamber after the sterilization procedure has ended. Additionally, the method may further comprise a step of averaging the image of the chemical indicator to create an averaged image such that the step of determining the color value of the image comprises determining the color value of the averaged image.

Determination of color values may be based on, e.g., the L*a*b* color model or a grayscale color model. As such, the color value may comprise an a* value such that, as determined by the inventors, the color-value threshold may comprise an a* value between approximately -9 and 9, e.g., approximately 0. Further, the a* value may comprise a value greater than about 48 during the step of placing the chemical indicator in the chamber. Where color values are expressed in grayscale, the color value may comprise a K% value such that, as determined by the inventors, the color-value threshold may comprise a K% value between approximately 50% and 60% black, e.g., about 54%. Further, the K% value may comprise a value greater than about 61% during the step of placing the chemical indicator in the chamber.

Another method disclosed herein for assessing an efficacy of a sterilization procedure performed by a sterilization apparatus comprising a chamber includes steps of placing a chemical indicator in the chamber, commencing a sterilization procedure, obtaining an image of the chemical indicator using a digital imaging device, determining a color value of the image of the chemical indicator, comparing the color value to a predetermined color-value threshold, determining that the color value has not passed the color-value threshold, waiting for a dwell time, introducing a volume of sterilant into the chamber, obtaining a subsequent image of the chemical indicator using the digital imaging device, determining a subsequent color value of the subsequent image of the chemical indicator, determining that the subsequent color value of the subsequent image of the chemical indicator has passed the color-value threshold, and automatically ending the sterilization procedure following a determination that the subsequent color value has passed the color-value threshold. The method may further comprise placing an instrument in a non-sterile state in the chamber proximate to the chemical indicator before commencing the sterilization procedure, automatically opening a door of the chamber after the sterilization procedure has ended, and removing the instrument in a sterile state from the chamber after the sterilization procedure has ended.

The method may further comprise averaging the image of the chemical indicator to create an averaged image such that the step of determining the color value of the image comprises determining the color value of the averaged image. Additionally, the method may also comprise averaging the subsequent image of the chemical indicator to create a subsequent averaged image such that the step of determining the subsequent color value of the subsequent image comprises determining the subsequent color value of the subsequent averaged image. The color value and the subsequent color value may comprise, e.g., a* values or grayscale values. As such, the color value may comprise an a* value such that, as determined by the inventors, the color-value threshold may comprise an a* value between approximately -9 and 9, e.g., approximately 0. Further, the a* value may comprise a value greater than about 48 during the step of placing the chemical indicator in the chamber. Where color values are expressed in grayscale, the color value may comprise an K% value such that, as determined by the inventors, the color-value threshold may comprise a K% value between approximately 50% and 60% black, e.g., about 54%. Further, the K% value may comprise a value greater than about 61% during the step of placing the chemical indicator in the chamber.

The present invention also relates to a method of operating a sterilization apparatus with the aid of a digital computer, the sterilization apparatus having a chamber and a digital imaging device. The method includes steps of providing the computer with a data base comprising color values according to the L*a*b* color space, placing a medical device in a non-sterile state and a chemical indicator into the chamber, placing a chemical indicator into the chamber proximate to the medical device, initiating an interval timer in the computer upon closure of the chamber for monitoring the elapsed time since the closure, repetitively obtaining an image of the chemical indicator using the digital imaging device, averaging each of the repeatedly obtained images of the chemical indicator, repetitively determining a color value for each of the averaged images, repetitively comparing each of the color values to a color-value threshold, repeatedly determining whether the color value has passed the color-value threshold, automatically ending the sterilization procedure following a determination that the color value has passed the color-value threshold, automatically opening the chamber, and removing the instruments in a sterile-state from the chamber.

In certain variations of this method, the color value may comprise an a* value or a K% value. As such, the color value may comprise an a* value such that, as determined by the inventors, the color-value threshold may comprise an a* value between approximately -9 and 9, e.g., approximately 0. Further, the a* value may comprise a value greater than about 48 during the step of placing the chemical indicator in the chamber. Where color values are expressed in grayscale, the color value may comprise an K% value such that, as determined by the inventors, the color-value threshold may comprise a K% value between approximately 50% and 60% black, e.g., about 54%. Further, the K% value may comprise a value greater than about 61% during the step of placing the chemical indicator in the chamber.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
Figure 1 depicts an assortment of biological indicators including chemical indicators that have been subject to different sterilization procedures ;
Figure 2 depicts a schematic of a sterilization system; and
Figure 3 depicts a flow chart reflecting an operating procedure for the sterilization system of Figure 2.

### MODES OF CARRYING OUT THE INVENTION

The following detailed description should be read with reference to the drawings, in which like elements in different drawings are identically numbered. The drawings, which are not necessarily to scale, depict selected embodiments and are not intended to limit the scope of the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

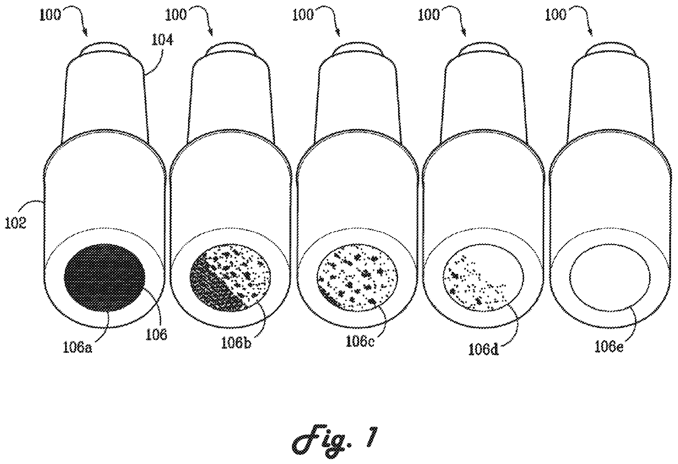

Figure 1 reflects a perspective view of five units of the STERRAD VELOCITY^{™} Biological Indicator 100 placed side by side. Each of these units includes a cap 102 and a vial 104. Affixed to each cap 102 is a class 5 chemical indicator 106. When a unit 100 is removed from its packaging, chemical indicator 106 is colored so as to indicate that the unit has never been in contact with a sterilant, such as hydrogen peroxide vapor. For example, the color red may be used to indicate that chemical indicator 106 has not been in contact with a sterilant. Although the commercial offering of the STERRAD VELOCITY^{™} Biological Indicator, which is used to describe the technological improvements herein, includes a chemical indicator that is sensitive to hydrogen peroxide, it should be understood that any class 5 chemical indicator may be used to practice the subject matter described herein, including independent of any biological indicator. Such class 5 chemical indicators may be sensitive to or change color when exposed to, e.g., steam, peracetic acid, ethylene oxide, or any other substance that may be used as a sterilant to kill microorganisms.

Biological indicator 100, including chemical indicator 106, may be exposed to a sterilant, e.g., hydrogen peroxide, typically during a sterilization procedure conducted by an automated sterilization system, which may be referred to as a sterilizer, such as ASP's STERRAD^{®} System, STERRAD^{®} NX System or STERRAD^{®} 100NX System. Upon exposure to the sterilant, the color of indicator 106 may change from a first color (e.g., red) that indicates indicator 106 has not been exposed to the sterilant to a second color (e.g., yellow) that indicates indicator 106 has been exposed to the sterilant . Typically the change between these two colors is not instantaneous, but may be gradual, depending on characteristics of the exposure, such as exposure time and sterilant concentration. Furthermore the transition between the two colors may not occur uniformly over the surface of indicator 106. For example, patches of coloration may occur during the transition from the first color to the second color. As shown, indicators 160a-e show different degrees of color transition based on differing amounts of sterilant exposure. Indicator 106a is uniformly the first color (shown in black) whereas indicator 106e is uniformly the second color (shown in white). Indicators 106b-d show irregular color patches that may occur during the color-change transition when exposure to the sterilant is less than the exposure necessary to achieve a complete transition from the first color to the second color.

A human user may reliably determine whether biological indicator 100 has been exposed to a sufficient or desired amount of sterilant, e.g., hydrogen peroxide, when the transition from the first color to the second color is complete. However, such a determination may not be reliable when the color transition is partial. For example, in Figure 1, the first color, e.g., red, is represented in black on chemical indicator 106, whereas the second color, e.g., yellow, is represented in white on chemical indicator 106. Chemical indictor 106a is uniformly the first color, indicating that it has not been exposed to a sterilant. Chemical indicator 106e is uniformly the second color, such that a human user should be able to determine form the chemical indicator alone that biological indicator 100 has been exposed to a sufficient or desired amount of sterilant, and to correspondingly determine that a sterilization procedure may have been efficacious. Chemical indicators 106b-d include various combinations of the first color and the second color, indicating that the transition from the first color (indicator 106a) to the second color (indicator 106e) was not complete, such that a human user might not be able to determine form the chemical indicator alone whether biological indicator 100 has been exposed to a sufficient or desired amount of sterilant. In these instances, a human user likely would determine that the sterilant exposure was insufficient such that the sterilization procedure was not efficacious even though it might have been. Applicant has thus developed a digital imaging and analysis technique that may be used improve the technology of assessing adequacy of a sterilization procedure by determining whether a partial color change, such as that of indicator 106d, indicates that biological indicator 100 has or has not been exposed to a sufficient or desired amount of sterilant, and whether a partial color change, such as that of indicator 106b or 106c, indicates that additional exposure to sterilant may be desired.

The digital imaging and analysis technique may be carried out according to various methods and variations, each of which includes at least the steps of obtaining an image of the chemical indicator, determining a color value of the image of the chemical indicator, comparing the color value to a color-value threshold, and determining that the color value passed the color-value threshold. The image of the chemical indicator may be obtained using a digital imaging device, such as a digital camera capable of taking a photograph of the chemical indicator. The image may then be input into a processor, e.g., a processor of the digital imaging device or of a digital computer, which may be independent from the digital imaging device. The processor may be configured to analyze the color(s) of the chemical indicator, e.g., by running thereon software, such as Adobe^{®} Photoshop^{®}. In particular, the color of the chemical indicator may be analyzed according to various color schemes, such as the L*a*b* color model ("Lab model") or a grayscale color model.

In the Lab model, colors are defined according to three perpendicular axes. The first axis is the L axis. Values on the lightness or L* axis indicate how much white to black may be present in an image or portion of an image, e.g., a pixel or collection of pixels. L* values typically range from 0 to 100, where 0 indicates darkest black and 100 indicates brightest white. Values on the a* axis indicate the amount of green to red, typically with -128 corresponding to pure green and 127 corresponding to pure red. Values on the b* axis indicate the amount of blue to yellow, typically with -128 corresponding to pure blue and 127 corresponding to pure yellow. In the grayscale model, colors are processed as though they comprise only black, white, or a combination thereof, similar to the L* value of the Lab model, except that in grayscale, 100% often refers to darkest black and 0% often refers to brightest white.

As such, the processor may process a color image of chemical indicator 106 and determine the L*, a*, and b* values in the Lab model. Alternatively or additionally, the processor may process the image to determine how black it is in the grayscale model. As noted, however, the chemical indicator 106 being analyzed may not have been subject to a sufficient amount of sterilant to change its color from the first color (e.g., red or black) to uniformly be the second color (e.g., yellow or white). In these instances, indicator 106 may include patches of the first color (e.g., a red corresponding to an a* of between about +48 and +127, which in grayscale correspond to a K% of about 61 to 100), patches of the second color (e.g., a yellow corresponding to an a* of between -128 and about 0, which in grayscale correspond to a K% of between 0 and about 54), and patches of colors in between the first color and second color (e.g., a light red or orange corresponding to an a* of between about 0 and about +48, which in grayscale correspond to a K% of between about 54 and about 76). The processor may thus compensate for the differing patches of color. One form of compensation may be called "blurring" or "averaging." For example, Adobe^{®} Photoshop^{®} includes a function called "Average Blur," which separately averages all of the L*, a*, and b* values, or grayscale percentage, for each pixel in an image or a portion of the image. Thus, for example, the a* value for each pixel may be summed and then divided by the total number of pixels to calculate an average a* value of the three patches.

This method of determining a color value for chemical indicator 106 may additionally be used to determine a color-value threshold upon which a determination of sufficient sterilant exposure may be based. This was accomplished by, first, subjecting a population of units of the STERRAD VELOCITY^{™} Biological Indicator to various sterilization cycles of the STERRAD^{®} NX, STERRAD^{®} 100NX, and STERRAD 100S such that sample sets of the units were exposed to different volumes of hydrogen peroxide for different times within the three systems. Specifically, in experiments performed by Applicant, five to ten units each were exposed to thirty nine combinations of hydrogen peroxide volume, exposure time, and system type, such that these units would either be incompletely sterilized or completely sterilized. In total, 340 units were tested. Second, for each biological indicator, color values (a* and grayscale) of each chemical indicator were then determined by: 1) taking a picture, i.e. digital image, of each chemical indicator using a digital camera; 2) opening each image in Adobe^{®} Photoshop^{®} CS6; 3) averaging each image of each chemical indicator using the Average Blur function; and 4) recording the a* value and grayscale value (referred to as K% in Adobe^{®} Photoshop^{®} CS6) reflected in the "Color" window when in, respectively, the L*a*b* color mode and grayscale color mode. Third, probability plots for a* and K% were created from data corresponding to the maximum injection volume for each of the cycles in each of the systems. These plots reflected that an a* value of less than 0 and a K% value of less than 54% would each provide a less than 1 in 10,000 chance that the chemical indicator was exposed to an insufficient amount of hydrogen peroxide to achieve sterilization. Accordingly, an a* of 0 and a K% value of 54% may be considered color-value thresholds such that a chemical indicator having an a* of less than approximately 0 or a K% of less than approximately 54% may be considered completely sterilized. If a lesser or greater reliability in the determination is desired, the color-value threshold may be adjusted. For example, if a greater reliability is desired, the color-value thresholds may be an a* of approximately -9 or a K% of approximately 50%. If a lesser reliability is desired, the color-value thresholds may be an a* of approximately 9 or a K% of approximately 60%.

Determination of the color value of chemical indicator 106, and whether the color value has passed the color-value threshold, may be performed after a sterilization cycle has been completed, such that comparison of the color value to the color-value threshold, and a sterilization determination based thereon, would also be performed after the sterilization cycle has been completed. A post-cycle sterilization determination of the chemical indicator may be an improvement over the current post-cycle sterilization determination based on assessment of a biological indicator because capturing an image of the chemical indicator, determining its color value, and comparing the color value to the color-value threshold may be accomplished more quickly than assessing a biological indicator. For example, assessment of the STERRAD VELOCITY^{™} Biological Indicator requires approximately thirty minutes, whereas assessment of the chemical indicator should require a five minutes or less to capture and analyze the image.

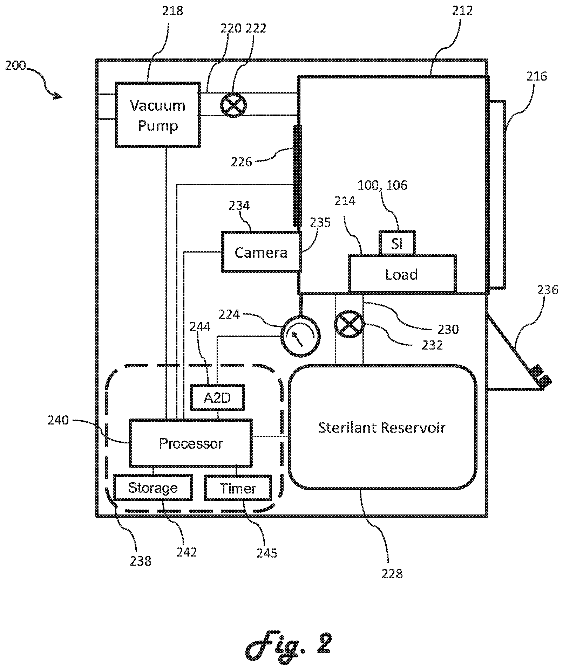

Determination of the color value of chemical indicator 106, and whether the color value has passed the color-value threshold, may also be performed repetitively during a sterilization cycle by a sterilizer, such as the sterilizer 200 depicted schematically in block diagram format in Figure 2. Sterilizer 200 comprises a chamber 212 having a load (pack) 214 of instruments therein to be sterilized. One or more sterilization indicators (i.e., biological indicator 100 having a chemical indicator 106, or a chemical indicator 106 separate from a biological indicator) may be disposed within chamber 212, such as placed upon or secured to load 214 as shown. The chamber 12 may be formed of any material that is sufficiently robust to handle pressures as low as approximately between 0.3 torr and 3 torr, and sufficiently inert to avoid reacting with or absorbing any sterilants introduced therein. Such materials may include aluminum and stainless steel. Chamber 212 may also include an openable and sealable barrier 216, such as a door, that may be opened to allow placement and removal of load 214 into chamber 212. The barrier should be sufficiently robust, and include a sufficiently robust seal, to withstand low pressures drawn within chamber 212 and avoid leaks between chamber 212 and the ambient environment. A vacuum pump 218 capable of reaching the desired operating pressure evacuates air and other gases, such as water vapor, from chamber 212. Vacuum pump 218 may include a hose or pipe 220 to connect it to chamber 212. Vacuum pump 218 may also include a valve 222, which may be open or closed to assist or prevent pressure changes in chamber 212. For example, when the valve is open and the vacuum pump is operational, the pressure in chamber 212 may be lowered. Alternatively, when the valve is open and the vacuum pump is not operational, the pressure in the chamber may be equalized to the ambient pressure. In other embodiments, a valve that is not part of vacuum pump 218 may be used to control whether chamber 212 has a pressure equal to the ambient pressure. A pressure monitor 224 monitors the pressure in chamber 212. Particularly suitable pressure monitors are capacitance manometers available from MKS Instruments. A heating element 226 may be used to heat the chamber 212. It may comprise separate elements bonded to the outside of the chamber 212 in locations sufficient to uniformly heat the chamber 212. A tank or reservoir 228 containing sterilant, which includes a hose or pipe 230, is connected to chamber 212. In some embodiments, tank 228 may further include a valve 232, which may be disposed between chamber 212 and tank 228 to control the flow of sterilant from tank 228 through hose 230 and into chamber 212. An imaging device, such as a camera 234, may be disposed alongside a viewing window 235 of chamber 212 for capturing images of chemical indicator 106. A power source and/or signal generator and an electrode (not shown) disposed within chamber 212 may be provided to create an electric field within chamber 212 between the electrode and the interior surface of chamber 212 to create a plasma therein. Creation of a plasma is useful for low temperature sterilization processes that use hydrogen peroxide gas. In these processes, the hydrogen peroxide gas may be excited to form a hydrogen peroxide plasma. Alternatively, another gas may be used to form the plasma, such as air, which may help lower hydrogen peroxide residuals upon the load to facilitate removal of hydrogen peroxide from chamber 212. Sterilization system 200 may also include a user interface 236, that may include output devices, such as a printer or display, and user-input devices, such as a keypad or touch screen.

A control system 238, such as a digital computer, controls the operation of sterilizer 200 and its various components. Control system 238 may employ one or more microprocessors 240. It may also employ a non-transitory storage medium 242, such as random access memory (RAM), a hard-disk drive, or flash memory, which can store data, such as color values (e.g., a data base comprising color values according to the Lab color model and a grayscale color model) and color-value thresholds. An analog to digital (A2D) converter 244 may be used to convert analog data to digital data if analog data, such as pressure data, is collected. A timer or clock circuit 245 keeps time. Control system 238 may further include software and/or logic by which microprocessor 240 may repetitively activate camera 234 to capture and store images of chemical indicator 106 in storage medium 242, analyze the images to determine a color value of the chemical indicator, compare color values to the color-value thresholds, and determine whether a color value has passed a color-value threshold. Alternatively, sterilizer 200 may include an input/output port such that an external digital computer may provide this software and/or logic, e.g., by including thereon software, such as Adobe^{®} Photoshop^{®} CS6, capable of determining color value, and outputting these values back to processor 240.

Processor 240 may be configured to automatically end a sterilization process upon determining that a color value has passed a color value-threshold. Alternatively or additionally, processor 240 may be configured to wait for a dwell time, automatically introduce a volume of sterilant from sterilant reservoir 228 into chamber 212, or both upon determining that a color value has not passed a color-value threshold

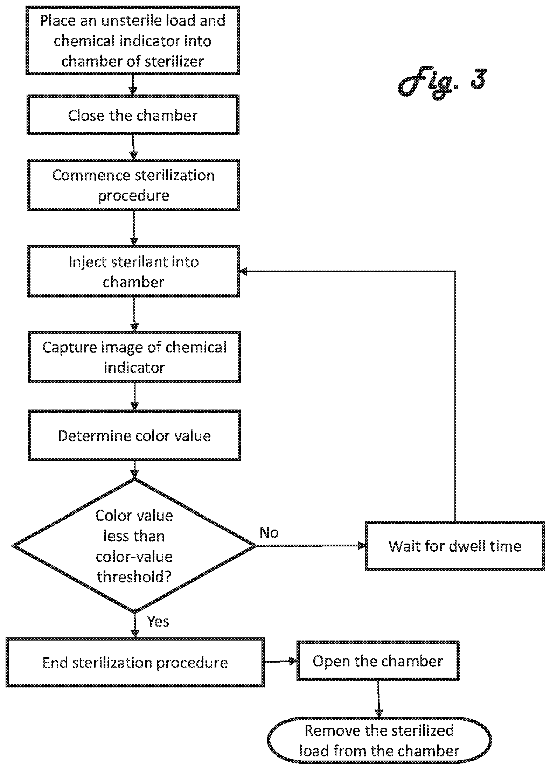

By virtue of the technology illustrated and described herein, and with reference to Figure 3, Applicant has devised a method and variations thereof for assessing an efficacy of a sterilization procedure performed by a sterilizer comprising a chamber. The method begins by placing a chemical indicator into the chamber. The chemical indicator may be affixed to a biological indicator or it may be independent of a biological indicator. For example, the chemical indicator may be placed into the chamber with or without a biological indicator. Preferably, a STERRAD VELOCITY^{™} Biological Indicator, which includes a chemical indicator, is placed into the chamber. The chemical indicator may be placed proximate to a load of instruments, e.g., medical devices, such as an endoscope, in the sterilization chamber. A door of the sterilizer may be closed such that the sterilization procedure may be commenced. During the sterilization procedure, a volume of a sterilant may be introduced, e.g., injected into the chamber to expose the chemical indicator and load to the sterilant. An imaging device, such as a camera, may then obtain an image of the chemical indicator. Next a color value of the image of the chemical indicator may be determined. This color value may be compared to a color-value threshold in order to determine whether the color value has passed the color-value threshold. If the color value has not passed the color value threshold, the processor may wait for a dwell time, e.g., approximately thirty seconds, approximately one minute, approximately two minutes, or approximately five minutes before continuing the procedure. Additionally or alternatively, the processor may cause an additional volume of sterilant to be introduced into the chamber after the dwell time. In certain variations of the method, the processor may not wait for a dwell time before injecting additional sterilant into the chamber, such that the dwell time may be considered zero seconds. After the dwell time or additional sterilant has been injected, a subsequent image may be obtained such that a subsequent color value of the subsequent image may be determined. This subsequent color value may then be compared to the color-value threshold in order to determine whether the subsequent color value has passed the color-value threshold. If not, the foregoing steps of waiting for a dwell time (which, again, is optional), introducing an additional volume of sterilant, obtaining a subsequent image, determining a subsequent color value, and determining whether the color value has passed the color-value threshold may be repeated until the subsequent color value has passed the color value threshold. Upon such determination, the sterilization procedure may be ended, e.g., automatically, by the processor. In certain variations of the method, the processor may automatically open a door of the chamber.

Typically, the load placed into the chamber with the chemical indicator comprises medical devices in a non-sterile state. Therefore, after the sterilization procedure has been ended, the medical devices may be removed from the chamber in a sterile state.

In further variations of the method, determination of the color values and subsequent color values may include a step of averaging the image of the chemical indicator to create an averaged image such that the steps of determining these color values includes determining the color values of the averaged images.

In some variations of the method, the color value comprises an a* value of the Lab color model. In these variations, the color-value threshold value comprises an a* value between approximately -9 and 9, such as approximately 0. In other variations of the method, the color value comprises a grayscale value. In these variations, the color-value threshold comprises a grayscale value between approximately 50% to approximately 60% black, such as approximately 54%.

Any of the examples or embodiments described herein may include various other features in addition to or in lieu of those described above. The teachings, expressions, embodiments, examples, etc., described herein should not be viewed in isolation relative to each other. Various suitable ways in which the teachings herein may be combined should be clear to those skilled in the art in view of the teachings herein.

## Claims

1. A method of assessing an efficacy of a sterilization procedure performed by a sterilization apparatus comprising a chamber (212), the method comprising:
placing a chemical indicator (106) in the chamber (212);
commencing a sterilization procedure;
obtaining an image of the chemical indicator (106) using a digital imaging device;
determining a color value of the image of the chemical indicator (106);
comparing the color value to a color-value threshold;
determining whether the color value passed the color-value threshold; and
automatically ending the sterilization procedure following a determination that the color value has passed the color-value threshold,
**characterized in that**
the method further comprises averaging the image of the chemical indicator (106) to create an averaged image such that the step of determining the color value of the image comprises determining the color value of the averaged image.

2. The method of claim 1, further comprising automatically opening a door of the chamber (212) after the sterilization procedure has ended.

3. The method of claim 2, further comprising:
placing an instrument in a non-sterile state in the chamber (212) proximate to the chemical indicator (106) before commencing the sterilization procedure; and
removing the instrument in a sterile state from the chamber (212) after the sterilization procedure has ended.

4. The method of claim 1, wherein the color value comprises an a* value.

5. The method of claim 4, wherein the color-value threshold comprises an a* value between approximately -9 and 9.

6. The method of claim 5, wherein the color-value threshold comprises an a* value of approximately 0.

7. The method of claim 1, wherein the color value comprises a grayscale value.

8. The method of claim 7, wherein the color-value threshold comprises a grayscale value that is between approximately 50% to approximately 60% black.

9. The method of claim 8, wherein the color-value threshold comprises a grayscale value of approximately 54%.

10. The method of any one of claims 1 to 9, the method further comprising:
before automatically ending the sterilization procedure, waiting for a dwell time after determining that the color value has not passed the color-value threshold;
introducing a volume of sterilant into the chamber (212);
obtaining a subsequent image of the chemical indicator (106) using the digital imaging device;
determining a subsequent color value of the subsequent image of the chemical indicator (106); and
determining that the subsequent color value of the subsequent image of the chemical indicator has passed the color-value threshold.

11. The method of claim 10, further comprising averaging the subsequent image of the chemical indicator to create a subsequent averaged image such that the step of determining the subsequent color value of the subsequent image comprises determining the subsequent color value of the subsequent averaged image.

12. An operating method for operating a sterilization apparatus using the method of any one of claims 1 to 11 with the aid of a digital computer, the sterilization apparatus having a chamber (212) and a digital imaging device, the operating method comprising:
providing the computer with a data base comprising color values according to the L*a*b* color space;
placing a medical device in a non-sterile state and a chemical indicator (106) into the chamber (212);
placing a chemical indicator (106) into the chamber (212) proximate to the medical device;
initiating an interval timer in the computer upon closure of the chamber (212) for monitoring the elapsed time since the closure;
repetitively obtaining an image of the chemical indicator (106) using the digital imaging device;
averaging each of the repeatedly obtained images of the chemical indicator (106);
repetitively determining a color value for each of the averaged images;
repetitively comparing each of the color values to a color-value threshold;
repeatedly determining whether the color value has passed the color-value threshold;
automatically ending the sterilization procedure following a determination that the color value has passed the color-value threshold;
automatically opening the chamber (212); and
removing the instruments in a sterile-state from the chamber (212).

## Patentansprüche

1. Verfahren zur Beurteilung der Wirksamkeit eines Sterilisationsverfahrens, das von einer Sterilisationsvorrichtung durchgeführt wird, die eine Kammer (212) umfasst, wobei das Verfahren umfasst:
Platzieren eines chemischen Indikators (106) in die Kammer (212);
Beginn eines Sterilisationsverfahrens;
Erhalten eines Bildes des chemischen Indikators (106) unter Verwendung einer digitalen Bildgebungsvorrichtung;
Bestimmen eines Farbwerts des Bildes des chemischen Indikators (106);
Vergleichen des Farbwerts mit einem Farbwert-Schwellenwert;
Bestimmen, ob der Farbwert den Farbwert-Schwellenwert überschritten hat; und
automatisches Beenden des Sterilisationsverfahrens nach einer Bestimmung, dass der Farbwert den Farbwert-Schwellenwert überschritten hat,
**dadurch gekennzeichnet, dass**
das Verfahren ferner das Mitteln des Bildes des chemischen Indikators (106) umfasst, um ein gemitteltes Bild zu erstellen, so dass der Schritt zum Bestimmen des Farbwerts des Bildes das Bestimmen des Farbwerts des gemittelten Bildes umfasst.

2. Verfahren nach Anspruch 1, ferner umfassend das automatische Öffnen einer Tür der Kammer (212) nach Beendigung des Sterilisationsverfahrens.

3. Verfahren nach Anspruch 2, ferner umfassend:
Platzieren eines Instruments in einem unsterilen Zustand in die Kammer (212) in der Nähe des chemischen Indikators (106) vor Beginn des Sterilisationsverfahrens; und
Entnehmen des Instruments in einem sterilen Zustand aus der Kammer (212) nach Beendigung des Sterilisationsverfahrens.

4. Verfahren nach Anspruch 1, wobei der Farbwert einen a*-Wert umfasst.

5. Verfahren nach Anspruch 4, wobei der Farbwert-Schwellenwert einen a*-Wert zwischen etwa -9 und 9 umfasst.

6. Verfahren nach Anspruch 5, wobei der Farbwert-Schwellenwert einen a*-Wert von etwa 0 umfasst.

7. Verfahren nach Anspruch 1, wobei der Farbwert einen Graustufenwert umfasst.

8. Verfahren nach Anspruch 7, wobei der Farbwert-Schwellenwert einen Graustufenwert umfasst, der zwischen etwa 50 % und etwa 60 % schwarz liegt.

9. Verfahren nach Anspruch 8, wobei der Farbwert-Schwellenwert einen Graustufenwert von etwa 54 % umfasst.

10. Verfahren nach einem der Ansprüche 1 bis 9, wobei das Verfahren ferner umfasst:
Abwarten einer Verweilzeit vor dem automatischen Beenden des Sterilisationsverfahrens, nachdem bestimmt wurde, dass der Farbwert den Farbwert-Schwellenwert nicht überschritten hat;
Einbringen einer Sterilisationsmittelmenge in die Kammer (212);
Erhalten eines anschließenden Bildes des chemischen Indikators (106) unter Verwendung der digitalen Bildgebungsvorrichtung;
Bestimmen eines anschließenden Farbwerts des anschließenden Bildes des chemischen Indikators (106); und
Bestimmen, dass der anschließende Farbwert des anschließenden Bildes des chemischen Indikators den Farbwert-Schwellenwert überschritten hat.

11. Verfahren nach Anspruch 10, ferner umfassend das Mitteln des anschließenden Bildes des chemischen Indikators, um ein anschließendes gemitteltes Bild zu erstellen, so dass der Schritt der Bestimmung des anschließenden Farbwerts des anschließenden Bildes das Bestimmen des anschließenden Farbwerts des anschließenden gemittelten Bildes umfasst.

12. Betriebsverfahren zum Betreiben einer Sterilisationsvorrichtung unter Verwendung des Verfahrens nach einem der Ansprüche 1 bis 11 mit Hilfe eines digitalen Computers, wobei die Sterilisationsvorrichtung eine Kammer (212) und eine digitale Bildgebungsvorrichtung aufweist, wobei das Betriebsverfahren umfasst:
Ausstatten des Computers mit einer Datenbank, die Farbwerte gemäß dem L*a*b*-Farbraum umfasst;
Platzieren einer medizinischen Vorrichtung in einem unsterilen Zustand und eines chemischen Indikators (106) in die Kammer (212);
Platzieren eines chemischen Indikators (106) in die Kammer (212) in die Nähe der medizinischen Vorrichtung;
Starten eines Intervalltimers im Computer beim Schließen der Kammer (212) zur Überwachung der seit dem Schließen abgelaufenen Zeit;
Wiederholtes Erhalten eines Bildes des chemischen Indikators (106) unter Verwendung der digitalen Bildgebungsvorrichtung;
Mitteln jedes der wiederholt erhaltenen Bilder des chemischen Indikators (106);
wiederholtes Bestimmen eines Farbwerts für jedes der gemittelten Bilder;
Wiederholtes Vergleichen jedes der Farbwerte mit einem Farbwert-Schwellenwert;
Wiederholtes Bestimmen, ob der Farbwert den Farbwert-Schwellenwert überschritten hat;
automatisches Beenden des Sterilisationsverfahrens nach einer Bestimmung, dass der Farbwert den Farbwert-Schwellenwert überschritten hat;
Automatisches Öffnen der Kammer (212); und
Entnehmen der Instrumente in einem sterilen Zustand aus der Kammer (212).

## Revendications

1. Procédé d'évaluation de l'efficacité d'une procédure de stérilisation effectuée par un appareil de stérilisation comprenant une chambre (212), la méthode comprenant :
placer un indicateur chimique (106) dans la chambre (212) ;
débuter une procédure de stérilisation ;
obtenir une image de l'indicateur chimique (106) à l'aide d'un dispositif d'imagerie numérique ;
déterminer une valeur de couleur de l'image de l'indicateur chimique (106) ;
comparer la valeur de la couleur à un seuil de valeur de la couleur ;
déterminer si la valeur de la couleur a dépassé le seuil de la valeur de la couleur ; et
mettre fin automatiquement à la procédure de stérilisation après avoir déterminé que la couleur a dépassé le seuil de la valeur de couleur,
**caractérisé par le fait que**
la méthode comprend en outre le calcul de la moyenne de l'image de l'indicateur chimique (106) pour créer une image moyennée de telle sorte que l'étape de détermination de la valeur de couleur de l'image comporte la détermination de la valeur de couleur de l'image moyennée.

2. Procédé selon la revendication 1, comprenant en outre l'ouverture automatique d'une porte de la chambre (212) après la fin de la procédure de stérilisation.

3. Procédé selon la revendication 2, comprenant en outre
placer un instrument non stérile dans la chambre (212) à proximité de l'indicateur chimique (106) avant de commencer la procédure de stérilisation ; et
retirer l'instrument stérile de la chambre (212) après la fin de la procédure de stérilisation.

4. Procédé selon la revendication 1, dans laquelle la valeur de couleur comprend une valeur a*.

5. Procédé selon la revendication 4, dans laquelle le seuil de valeur de couleur comprend une valeur a* comprise approximativement entre -9 et 9.

6. Procédé selon la revendication 5, dans laquelle le seuil de la valeur de couleur comprend une valeur a* d'environ 0.

7. Procédé selon la revendication 1, dans laquelle la valeur de couleur comprend une valeur de niveau de gris.

8. Procédé selon la revendication 7, dans laquelle le seuil de la valeur de couleur comprend une valeur de niveau de gris comprise entre environ 50 % et environ 60 % de noir.

9. Procédé selon la revendication 8, dans laquelle le seuil de la valeur de couleur comprend une valeur de niveau de gris d'environ 54 %.

10. Procédé selon l'une des revendications 1 à 9, comprenant en outre :
avant de mettre fin automatiquement à la procédure de stérilisation, attendre un temps d'arrêt après avoir déterminé que la valeur de couleur n'a pas dépassé le seuil de la valeur de la couleur ;
introduire un volume de stérilisant dans la chambre (212) ;
obtenir une image ultérieure de l'indicateur chimique (106) à l'aide du dispositif d'imagerie numérique ;
déterminer une valeur de couleur ultérieure de l'image ultérieure de l'indicateur chimique (106) ; et
déterminer que la valeur de couleur subséquente de l'image subséquente de l'indicateur chimique a dépassé le seuil de la valeur de couleur.

11. Procédé selon la revendication 10, comprenant en outre le calcul de la moyenne de l'image ultérieure de l'indicateur chimique pour créer une image moyenne ultérieure de telle sorte que l'étape de détermination de la valeur de couleur ultérieure de l'image ultérieure comprend la détermination de la valeur de couleur ultérieure de l'image moyenne ultérieure.

12. Procédé de fonctionnement d'un appareil de stérilisation utilisant la méthode selon l'une quelconque des revendications 1 à 11 à l'aide d'un ordinateur numérique, l'appareil de stérilisation ayant une chambre (212) et un dispositif d'imagerie numérique, la méthode de fonctionnement comprenant :
fournir à l'ordinateur une base de données comprenant des valeurs de couleur selon l'espace colorimétrique L*a*b* ;
placer un dispositif médical non stérile et un indicateur chimique (106) dans la chambre (212) ;
placer un indicateur chimique (106) dans la chambre (212) à proximité du dispositif médical ;
déclencher une minuterie dans l'ordinateur lors de la fermeture de la chambre (212) pour suivre le temps écoulé depuis la fermeture ;
obtenir de manière répétitive une image de l'indicateur chimique (106) à l'aide du dispositif d'imagerie numérique ;
calculer la moyenne de chacune des images de l'indicateur chimique (106) obtenues de manière répétitive ;
déterminer de manière répétitive une valeur de couleur pour chacune des images moyennées ;
comparer de manière répétitive chacune des valeurs de couleur à un seuil de valeur de couleur ;
déterminer de manière répétitive si la valeur de la couleur a dépassé le seuil de la valeur de couleur ;
mettre fin automatiquement à la procédure de stérilisation après avoir déterminé que la couleur a dépassé le seuil de la valeur de couleur ;
ouvrir automatiquement la chambre (212) ; et
retirer les instruments stériles de la chambre (212).
